(19) Europäisches Patentamt European Patent Office Office européen des brevets

(11) **EP 4 512 316 A1**

(12) **EUROPEAN PATENT APPLICATION**
published in accordance with Art. 153(4) EPC

(43) Date of publication:
**26.02.2025 Bulletin 2025/09**

(21) Application number: **23822225.1**

(22) Date of filing: **26.06.2023**

(51) International Patent Classification (IPC):
***A61B 5/021*** (2006.01)

(86) International application number:
**PCT/CN2023/102523**

(87) International publication number:
**WO 2025/000204 (02.01.2025 Gazette 2025/01)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Shenzhen Goodix Technology Co., Ltd.
Shenzhen, Guangdong 518045 (CN)**

(72) Inventors:
• **CHENG, Shuqing
  Shenzhen, Guangdong 518045 (CN)**
• **QIU, Fangfang
  Shenzhen, Guangdong 518045 (CN)**

(74) Representative: **Kraus & Lederer PartGmbB
Thomas-Wimmer-Ring 15
80539 München (DE)**

(54) **BIOMETRIC INFORMATION MEASUREMENT APPARATUS AND ELECTRONIC DEVICE**

(57) Disclosed herein is an apparatus for detecting biometric information, including a finger stall, a pulse wave sensor, an analog front end, and a microcontroller unit. The finger stall includes a mechanical structure or a gasbag. The pulse wave sensor includes a light emitter and a photodiode. The analog front end is electrically connected to the pulse wave sensor, and is configured to receive an electrical signal and output an analog front end signal. The microcontroller unit is electrically connected to the analog front end, and is configured to receive the analog front end signal and output the biometric information, the biometric information including a blood pressure value. The apparatus for detecting biometric information can enable a miniaturization of a blood pressure measuring apparatus, facilitate offering portability for users, and achieve blood pressure monitoring for the users in a non-household environment.

FIG. 2

EP 4 512 316 A1

**Description**

## TECHNICAL FIELD

The present disclosure relates to the technical field of wearable devices, and more specifically relates to an apparatus for detecting biometric information and an electronic device.

## BACKGROUND

At present, a mercury sphygmomanometer is a golden standard in the field of blood pressure measurement. The mercury sphygmomanometer requires a user to have certain professional knowledge, and can be dynamically regulated by the user according to actual situation of a testee, so that its test result is accurate and it is applicable to a wide range of people. Electronic sphygmomanometers available on the current market may not need to be used and operated by professional personnel, but the electronic sphygmomanometers cannot meet the needs of hypertension groups for real-time blood pressure management because their overall size is large and they are generally suitable for placement in households and are not portable.

Therefore, how to miniaturize a blood pressure measuring apparatus, so that it facilitates offering portability for users, and achieves blood pressure monitoring in a non-household environment for the users, is a technical problem in urgent need of solution.

## SUMMARY

A first aspect of embodiments of the present disclosure provides an apparatus for detecting biometric information, including:

a finger stall comprising a mechanical structure or a gasbag, where the mechanical structure or the gasbag is configured to fix the finger stall on a finger of a user;
a pulse wave sensor comprising a light emitter and a photodiode, where the light emitter is configured to emit a light signal which reaches the photodiode after being reflected or transmitted by a to-be-detected object, and the photodiode is configured to convert the received light signal into an electrical signal;
an analog front end electrically connected to the pulse wave sensor and configured to receive the electrical signal and output an analog front end signal; and
a microcontroller unit electrically connected to the analog front end and configured to receive the analog front end signal and output the biometric information, the biometric information including a blood pressure value.

In a possible embodiment, the apparatus further includes a pressure detector electrically connected to the analog front end.

In a possible embodiment, the finger stall is a two-end through type finger stall with an annular cross section.

In a possible embodiment, the finger stall is a non-through type;

the non-through type finger stall is only provided with an opening at one end; and
a cross section of the non-through type finger stall is one of rectangle, circle, ellipse, and polygon, and the non-through type finger stall has an internal space for accommodating the finger of the user.

In a possible embodiment, the finger stall further includes an air conduit connected to the gasbag.

In a possible embodiment, the mechanical structure includes a spring structure, where the spring structure includes a spring body and a connecting plate; and

the connecting plate is connected to one end of the spring body, another end of the spring body is connected to the finger stall, and the spring structure is arranged on one side inside the finger stall close to the opening.

In a possible embodiment, the spring body includes a first spring body and a second spring body, and the connecting plate includes a first connecting plate and a second connecting plate; and

the first connecting plate is connected to the first spring body, the second connecting plate is connected to the second spring body, the light emitter is arranged on the first connecting plate, and the photodiode is arranged on the second connecting plate.

In a possible embodiment, the mechanical structure or the gasbag is arranged on one side inside the finger stall close to the opening.

In a possible embodiment, the mechanical structure includes a motor structure, where the motor structure includes a motor and a connecting plate, the connecting plate is connected to the motor, the motor is configured to control movement of the connecting plate, and the motor structure is arranged on one side inside the finger stall close to the opening.

In a possible embodiment, the biometric information further includes at least one of a heart rate value and a blood oxygen value.

A second aspect of the embodiments of the present disclosure provides an electronic device, including the apparatus for detecting biometric information provided in the first aspect of the embodiments of the present disclosure and a display screen arranged on an outer surface of the apparatus, where the display screen is configured to display the biometric information.

In a possible embodiment, the electronic device includes a smart ring, a finger clip-type blood pressure meter, a finger clip-type blood pressure oximeter, and a finger clip-type multi-parameter meter.

Embodiments of the present disclosure provide an apparatus for detecting biometric information that can measure blood pressure based on a finger, and complete accurate blood pressure measurement in a small volume, thereby miniaturizing a blood pressure measuring apparatus, and facilitating offering portability for users.

The embodiments of the present disclosure further provide an electronic device that can facilitate quickly and conveniently monitoring real-time blood pressure of hypertension users in a non-household environment, and improving the user experience.

**BRIEF DESCRIPTION OF DRAWINGS**

FIG. 1 is a schematic diagram of a finger blood vessel changing from diastole to systole, and then returning to diastole.
FIG. 2 is a schematic structural diagram of an apparatus for detecting biometric information provided in an embodiment of the present disclosure.
FIG. 3 is a schematic structural diagram of another apparatus for detecting biometric information provided in an embodiment of the present disclosure.
FIG. 4 is a schematic structural diagram of still another apparatus for detecting biometric information provided in an embodiment of the present disclosure.
FIG. 5 is a PPG waveform amplitude curve of a blood vessel in a process of changing from complete systole to recovery of diastole.
FIG. 6 is a schematic diagram of computing blood pressure by double Gaussian fitting.
FIG. 7 is a schematic diagram of a blood pressure detection result detected at a third knuckle of a finger via an apparatus for detecting biometric information provided in an embodiment of the present disclosure.
FIG. 8 is a schematic diagram of a blood pressure detection result detected at a second knuckle of a finger via an apparatus for detecting biometric information provided in an embodiment of the present disclosure.
FIG. 9 is a structural block diagram of an electronic device provided in an embodiment of the present disclosure.
FIG. 10 is a schematic diagram of an electronic device provided in an embodiment of the present disclosure.

**DESCRIPTION OF EMBODIMENTS**

Technical solutions of embodiments of the present disclosure will be clearly and completely described below with reference to the drawings.

The terms used in the present disclosure are intended merely to describe particular embodiments, and are not intended to limit the present disclosure. The singular forms of "a" and "the" used in the present disclosure and the appended claims are also intended to include plural forms, unless the context clearly indicates other meanings.

In addition, the terms such as "first" and "second" are only used for distinguishing between similar objects, but cannot be understood as indicating or implying relative importance or implicitly indicating the number of indicated technical features. Thus, features defined with "first," "second," or the like may explicitly or implicitly include one or more of the features.

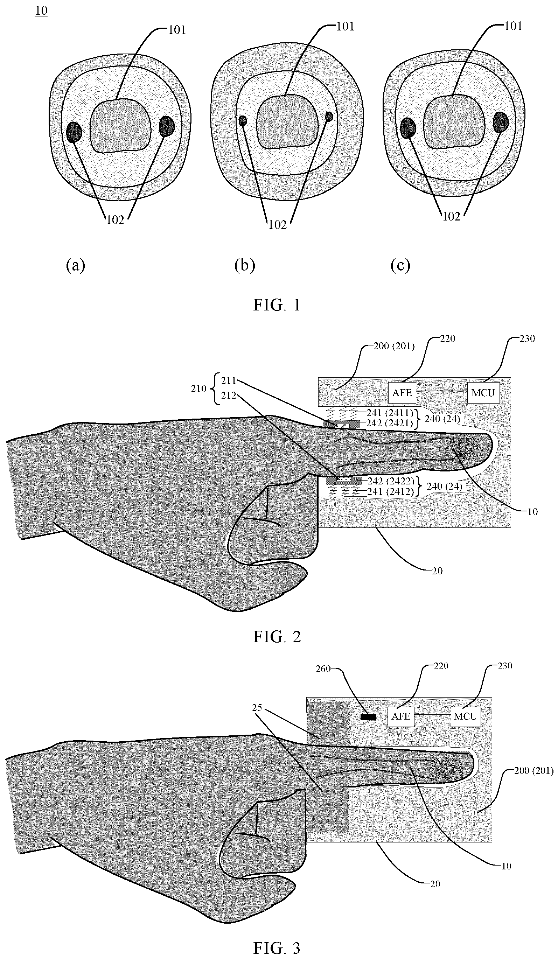

FIG. 1 shows a schematic diagram of a finger blood vessel changing from diastole to systole, and then returning to diastole.

As shown in FIG. 1(a), a finger 10 includes a bone 101 and a blood vessel 102. In the case when the finger 10 is in a normal resting state, the blood vessel 102 is in a diastolic state. When a pressure is applied to the finger 10, the blood vessel 102 on either side of the finger 10 will gradually change from the diastolic state to a systolic state, as shown in FIG. 1(b). In this case, blood flow in the blood vessel 102 will decrease to a nearly cutoff state. Then, in the case when the pressure applied to the finger 10 is gradually released, as shown in FIG. 1(c), the blood vessel 102 will gradually restore from the systolic state to the diastolic state.

In the case when the blood vessel 102 is systolic or the blood vessel 102 is diastolic, a pressure value and a pressure pulse wave or a photoelectric pulse wave of the blood vessel 102 are recorded synchronously.

A waveform of the pressure pulse wave and a waveform of the photoelectric pulse wave have similar morphological characteristics. The pressure pulse wave and the photoelectric pulse wave are actually two different manifestations of a same arterial periodic pulsation process, and therefore both essentially reflect a functional state of the cardiovascular system. Therefore, characteristics of pressure pulse wave or photoelectric pulse wave signals may be analyzed and studied, to extract physiological and pathological information contained therein, thus providing assistance for early diagnosis and prevention of cardiovascular system-related diseases.

The pressure pulse wave is generally detected and acquired at superficial arteries such as radial artery, carotid artery, or femoral artery. A change curve of arterial pressure over time is photoplethysmographized using a pressure detector to obtain the waveform of the pressure pulse wave. The photoelectric pulse wave is generally detected and acquired using a photoelectric sensor, is therefore often referred to as photoelectric volume pulse wave, and is usually detected and obtained by photo plethysmography (PPG). A change curve of blood volume over time photoplethysmographized using this method is called the waveform of the photoelectric pulse wave, and is also abbreviated as PPG waveform below.

Relevant features, such as a peak value of the PPG waveform, are extracted based on the waveform of the pressure pulse wave or the photoelectric pulse wave during systole or diastole, to plot an envelope curve. According to the principle of oscillography, a maximum amplitude is the Mean Blood Pressure (MBP), and then, a systolic blood pressure (SBP) and a diastolic blood pressure (DBP) can be obtained by conventional double Gaussian fitting, envelope bathmometry, or deep learning algorithm.

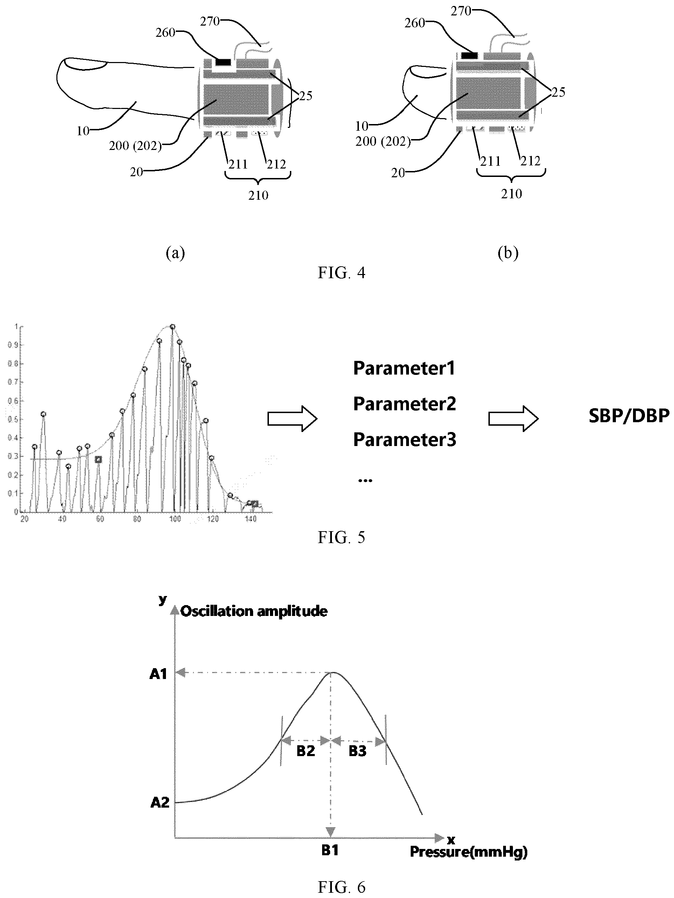

Specifically, FIG. 2 shows a schematic structural diagram of an apparatus for detecting biometric information provided in an embodiment of the present disclosure, FIG. 3 shows a schematic structural diagram of another apparatus for detecting biometric information provided in an embodiment of the present disclosure, and FIG. 4 shows a schematic structural diagram of still another apparatus for detecting biometric information provided in an embodiment of the present disclosure.

As shown in FIG. 2, FIG. 3, and FIG. 4, embodiments of the present disclosure provide an apparatus 20 for detecting biometric information. The apparatus 20 includes:

a finger stall 200, the finger stall 200 including a mechanical structure 24 or a gasbag 25, where the mechanical structure 24 or the gasbag 25 is configured to fix the finger stall 200 on a finger 10 of a user.

Optionally, referring to FIG. 4, the finger stall 200 is a two-end through type, and the two-end through type finger stall 202 has an annular cross section, including a circular annular shape, a triangular annular shape, a square annular shape, an elliptical annular shape, and a wavy annular shape. The finger stall 202 in FIG. 4 is similar to a ring. The user can wear the two-end through type finger stall 202 on a third knuckle (as shown in FIG. 4(a)) or a second knuckle (as shown in FIG. 4(b)) of the finger 10, to conveniently and quickly achieve blood pressure detection.

Optionally, referring to FIG. 2 and FIG. 3, the finger stall 200 is a non-through type. That is, the non-through type finger stall 201 is only provided with an opening at one end, a cross section of the non-through type finger stall 201 is one of rectangle, circle, ellipse, and polygon, and the non-through type finger stall 201 has an internal space for accommodating the finger 10 of the user. In daily use, the non-through type finger stall 201 is less affected by environmental light, thereby not only achieving highly accurate blood pressure detection, but also satisfying the requirements for convenience and high efficiency.

In the embodiment of the present disclosure, the two-end through type finger stall 202 and the non-through type finger stall 201 may each include the mechanical structure 24 or the gasbag 25.

Optionally, the mechanical structure 24 or the gasbag 25 is arranged on one side inside the finger stall 200 close to the opening.

When the non-through type finger stall 201 is worn on the finger 10 of the user, the opening side of the non-through type finger stall 201 is close to the third knuckle of the finger 10, and the mechanical structure 24 or the gasbag 25 is arranged on one side inside the non-through type finger stall 201 close to the opening, to collect a waveform of a pressure pulse wave or a photoelectric pulse wave at the third knuckle.

As an optional embodiment, referring to FIG. 3 and FIG. 4, the finger stall 200 includes the gasbag 25 and an air conduit 270, where the air conduit 270 is connected to the gasbag 25, and the air conduit 270 can inflate and deflate the gasbag 25.

As another optional embodiment, the finger stall 200 includes the mechanical structure 24, where the mechanical structure 24 is configured to fix the finger stall 200 on the finger 10 of the user. The mechanical structure 24 includes a spring structure 240, where the spring structure 240 includes a spring body 241 and a connecting plate 242. The connecting plate 242 is connected to one end of the spring body 241, another end of the spring body 241 is connected to the finger stall 200, and the spring structure 240 is arranged on one side inside the finger stall 200 close to the opening.

Specifically, as an optional embodiment, referring to FIG. 2, the spring body 241 includes a first spring body 2411 and a second spring body 2412, and the connecting plate 242 includes a first connecting plate 2421 and a second connecting plate 2422; and

the first connecting plate 2421 is connected to the first spring body 2411, the second connecting plate 2422 is connected to the second spring body 2412, a light emitter 211 is arranged on the first connecting plate 2421, and a photodiode 212 is arranged on the second connecting plate 2422.

As another optional embodiment, the mechanical structure 24 includes a motor structure, where the motor structure includes a motor and a connecting plate, the connecting plate is connected to the motor, the motor is configured to control movement of the connecting plate, and the motor structure is arranged on one side inside the finger stall 200 close to the opening. The motor can control the connecting plate to perform systolic and diastolic operations on the blood vessel 102.

A pulse wave sensor 210, the pulse wave sensor 210 including the light emitter 211 and the photodiode 212, where the light emitter 211 is configured to emit a light signal which reaches the photodiode 212 after being reflected or transmitted by a to-be-detected object, and the photodiode 212 is configured to convert the received light signal into an electrical signal.

Specifically, referring to the pulse wave sensor 210 in FIG. 2, in the transmission-type pulse wave sensor 210, the light signal emitted from the light emitter 211 is received by the photodiode 212 through muscles, bones, veins, and other connecting tissues of the finger 10.

Specifically, referring to the pulse wave sensor 210 in FIG. 4, in the reflection-type pulse wave sensor 210, the light signal emitted from the light emitter 211 is reflected to the photodiode 212 through skin tissues of the finger 10.

An analog front end (AFE) 220, the analog front end 220 being electrically connected to the pulse wave sensor 210, the analog front end 220 being configured to receive the electrical signal and output an analog front end signal.

Specifically, the analog front end 220 can perform, e.g., amplification, filtering, and analog-to-digital (AD) conversion on the electrical signal sensed by the pulse wave sensor 210, and the processed signal is the analog front end signal.

A microcontroller unit (MCU) 230, the microcontroller unit 230 being electrically connected to the analog front end 220, the microcontroller unit 230 being configured to receive the analog front end signal and output the biometric information, the biometric information including a blood pressure value.

Optionally, during blood pressure detection, the microcontroller unit (MCU) can process the analog front end signal by conventional double Gaussian fitting, envelope bathmometry, or deep learning algorithm, and then obtain a blood pressure value including a systolic blood pressure (SBP) and a diastolic blood pressure (DBP).

As an optional embodiment, the biometric information further includes at least one of a heart rate value and a blood oxygen value. The pulse wave sensor 210 can be used for both blood pressure detection, and heart rate and blood oxygen detection, thereby achieving multiplexing of the pulse wave sensor 210, simplifying the structure of the apparatus 20, and saving costs.

Optionally, referring to FIG. 3 and FIG. 4, the apparatus 20 further comprises a pressure detector 260, where the pressure detector 260 is electrically connected to the analog front end 220.

As an optional embodiment, the pressure detector 260 can be configured to measure the pressure pulse wave, and then compute the blood pressure value based on the pressure pulse wave, while the pulse wave sensor 210 will be configured to measure the heart rate value and the blood oxygen value.

As another optional embodiment, the pressure detector 260 and the pulse wave sensor 210 can be combined to measure the blood pressure value, and two initial blood pressure values can be computed based on the pressure pulse wave measured by the pressure detector 260 and the photoelectric pulse wave measured by the pulse wave sensor 210, respectively. Then, the two initial blood pressure values can be processed, e.g., by averaging to obtain a target blood pressure value. In the present embodiment, the blood pressure value is computed using both the pressure detector 260 and the pulse wave sensor 210, thereby improving the accuracy of the computation result of the blood pressure value.

It should be noted that the pressure detector 260 not only can be applied to the apparatus in FIG. 3 and FIG. 4, but also can be applied to the apparatus 20 in FIG. 2. The scope of application of the pressure detector 260 is not limited in the embodiments of the present disclosure.

FIG. 5 is a PPG waveform amplitude curve of a blood vessel in a process of changing from complete systole to recovery of diastole.

Specifically, referring to both FIG. 1 and FIG. 5, taking the blood pressure measurement by oscillography as an example, the photoelectric pulse wave amplitude curve is acquired by applying a pressure to the finger 10 to achieve a process of blocking the blood vessel 102. Multi-dimensional curve features are acquired by curve fitting to acquire a final blood pressure result, namely the systolic blood pressure (SBP) and the diastolic blood pressure (DBP) in FIG. 5, based on curve fitting parameters (Parameter 1, Parameter 2, Parameter 3, ... in FIG. 5). In the PPG waveform amplitude curve shown in FIG. 5, the abscissa is pressure, and the ordinate is the PPG waveform amplitude.

General computing method of the oscillography is that: a pressure corresponding to maximum change of the blood vessel volume is the mean blood pressure (MBP), and then an internal relationship between the systolic blood pressure (SBP), the diastolic blood pressure (DBP), and the mean blood pressure (MBP) is determined based on envelope curve features, to finally obtain the blood pressure result.

FIG. 6 is a schematic diagram of computing blood pressure by double Gaussian fitting. With reference to FIG. 5 and FIG. 6, the method for computing the blood pressure result is as follows:
first, the envelope curve is fitted using a double Gaussian function:

$$x < B1, \; y = A2 + (A1 - A2)e^{-\frac{1}{2}(\frac{x-B1}{B2})^2}$$

$$x \geq B1, \; y = A1e^{-\frac{1}{2}(\frac{x-B1}{B2})^2}$$

A1 and B1 are an ordinate and an abscissa of a peak point of the envelope curve respectively, A2 is an ordinate of an intersection point of the envelope curve and the y-axis, B2 is a difference between an abscissa of a point that is located to the left of the peak point and is half the height of the peak point on the envelope curve (that can be called the left half height point) and the abscissa of the peak point, and B3 is a difference between an abscissa of a point that is located to the right of the peak point and is half the height of the peak point on the envelope curve (that can be called the right half height point) and the abscissa of the peak point.

Then, after parameters of the envelope curve are obtained, the diastolic blood pressure (DBP), the systolic blood pressure (SBP), and the mean blood pressure (MBP) are computed as per the following formula:

$$DBP = 0.65B1 - 1.54\frac{A2}{A1}B2 + 22.6$$

$$MBP = 0.68B1 - 1.55\frac{A2}{A1}B2 + 33.2$$

$$SBP = 2.5 * MBP - 1.6 * DBP$$

The blood pressure can be computed not only by the above-mentioned double Gaussian fitting, but also selectively by envelope bathmometry.

Specifically, the principle of the envelope bathmometry is that: an artery has highest elasticity under zero transmural pressure conditions, and two inflection points are located to the left and to the right of the peak point of the envelope curve of the pressure pulse wave. Cuff pressures corresponding to the two inflection points are the systolic blood pressure (SBP) and the diastolic blood pressure (DBP), respectively. The inflection points correspond to largest pressure changes, so that an absolute value of a first derivative of the corresponding envelope curve is a maximum value. In this case, a value of a second derivative of the corresponding envelope curve should be 0. Therefore, the two inflection points to the left and to the right of the peak point of the envelope curve can be found by computing the value of the first derivative or the value of the second derivative of the envelope curve of the pressure pulse wave, thereby computing the systolic blood pressure (SBP) and the diastolic blood pressure (DBP) respectively.

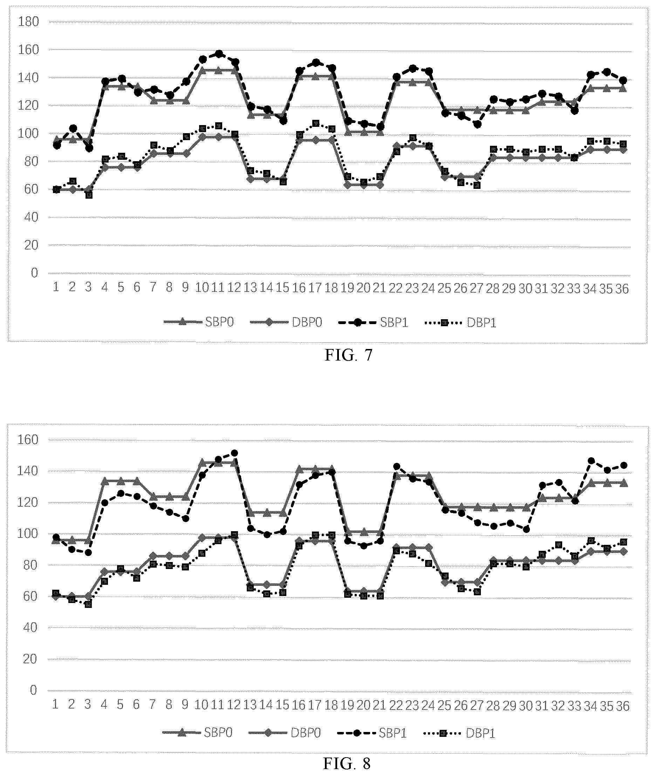

FIG. 7 and FIG. 8 are a schematic diagram of a blood pressure detection result detected at a third knuckle of a finger via an apparatus for detecting biometric information provided in an embodiment of the present disclosure and a schematic diagram of a blood pressure detection result detected at a second knuckle of a finger via an apparatus for detecting biometric information provided in an embodiment of the present disclosure, respectively.

In FIG. 7 and FIG. 8, the abscissas represent different tested objects, while the ordinates represent sizes of blood pressure values (mmHg).

The SBP0 and DBP0 represent real values of the systolic blood pressure and the diastolic blood pressure recorded using a mercury stethoscope, while SBP1 and DBP1 represent values of the systolic blood pressure and the diastolic blood pressure computed by collecting a pressure pulse wave at a fingertip.

As can be seen from FIG. 7 and FIG. 8, the blood pressure detection results at the third knuckle and the second knuckle are very close to the blood pressure detection results recorded using a mercury stethoscope. Therefore, it is feasible to perform blood pressure detection at the third knuckle and the second knuckle.

In Table 1 below, blood pressure measurement is performed on 25 subjects at different sites such as the arm, wrist, third knuckle, and second knuckle by collecting pressure pulse waves via oscillography, by envelope fitting via extraction of peaks of the pressure pulse waves, and finally by computing a correlation coefficient among the envelope peak (EP) and the systolic blood pressure (SBP), the diastolic blood pressure (DBP), and the mean arterial pressure (MAP).

Table 1

| Position | Normal blood pressure (15 people) | | | Hypertension (10 people) | | |
|---|---|---|---|---|---|---|
| | SBP vs. EP | DBP vs. EP | MAP vs. EP | SBP vs. EP | DBP vs. EP | MAP vs. EP |
| Arm | 0.9682 | 0.9845 | 0.9799 | 0.9492 | 0.8307 | 0.9161 |
| Wrist | 0.8655 | 0.9550 | 0.9625 | 0.7405 | 0.7444 | 0.7627 |
| Third knuckle | 0.8332 | 0.9013 | 0.8975 | 0.6945 | 0.8776 | 0.7203 |
| Second knuckle | 0.8181 | 0.8006 | 0.8338 | 0.6922 | 0.8924 | 0.8226 |

As can be seen from Table 1, the correlation coefficient among the systolic blood pressure (SBP), the diastolic blood pressure (DBP), and the mean arterial pressure (MAP) measured at the arm, wrist, third knuckle, and second knuckle and the envelope peak EP is strongly correlated with blood pressures at the third knuckle and the second knuckle, no matter for subjects with normal blood pressure or for subjects with hypertension.

The accuracy evaluation results of the blood pressure data measured at the third knuckle and the second knuckle are presented in Table 2 below, respectively.

Table 2

| Test site | ISO standard (SD<8, MD<5) | | | |
|---|---|---|---|---|
| | SBP_MD | SBP_SD | DBP_MD | DBP_SD |
| Third knuckle | -4.44 | 5.67 | -3.89 | 4.16 |
| Second knuckle | 4.17 | 7.73 | 1.53 | 4.64 |

The MD represents a median deviation (MD), and SD represents a standard deviation (SD).

The accuracy evaluation results of the blood pressure data measured at the third knuckle and the second knuckle can be seen from Table 2. Specifically, an absolute value of a median deviation (SBP_MD) of the systolic blood pressure (SBP) and an absolute value of a median deviation (DBP_MD) of the diastolic blood pressure (DBP) are each less than 5, and an absolute value of a standard deviation (SBP_SD) of the systolic blood pressure (SBP) and an absolute value of a standard deviation (DBP_SD) of the diastolic blood pressure (DBP) are each less than 8, complying with the ISO medical standard.

The apparatus 20 for detecting biometric information provided in the embodiments of the present disclosure measures blood pressure at the third knuckle and the second knuckle, and the accuracy of the obtained blood pressure data is up to standard.

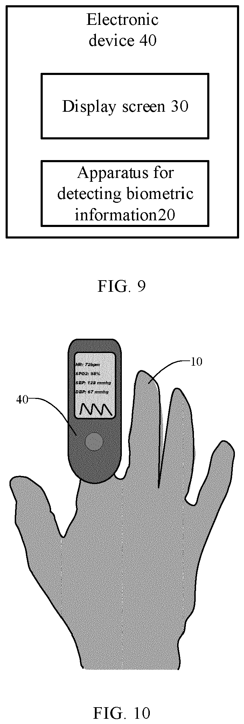

FIG. 9 is a structural block diagram of an electronic device provided in an embodiment of the present disclosure.

As shown in FIG. 9, the embodiment of the present disclosure further provides an electronic device 40. The electronic device 40 includes the apparatus 20 for detecting biometric information provided in the above embodiments of the present disclosure and a display screen 30, where the display screen 30 is arranged on an outer surface of the apparatus 20, and the display screen 30 is configured to display the biometric information.

Specifically, the biometric information displayed on the display screen 30 includes a blood pressure value, a blood oxygen value, and a heart rate value. The display screen 30 can display the blood pressure value separately, or can display the blood pressure value, the blood oxygen value, and the heart rate value simultaneously.

Optionally, the wearable device further includes a portable blood pressure measuring device, such as a smart ring, a finger clip-type blood pressure meter, a finger clip-type blood pressure oximeter, and a finger clip-type multi-parameter meter.

FIG. 10 is a schematic diagram of an electronic device provided in an embodiment of the present disclosure.

As shown in FIG. 10, the electronic device 40 is a multifunctional finger clip-type blood pressure oximeter, which can measure the biometric information, such as a heart rate value, a blood oxygen value, a heart rate variability (HRV), and a blood pressure value.

The electronic device 40 in the embodiments of the present disclosure can facilitate quickly and conveniently monitoring real-time blood pressure of hypertension users in a non-household environment, and improve the user experience.

The preferred embodiments of the present disclosure are described in detail above with reference to the drawings. However, the present disclosure is not limited to the specific details of the above-mentioned embodiments. Within the scope of the technical concept of the present disclosure, multiple simple modifications can be made to the technical solutions of the present disclosure, and the simple modifications are all encompassed within the scope of protection of the present disclosure.

**Claims**

1. An apparatus for detecting biometric information, comprising:

   a finger stall comprising a mechanical structure or a gasbag, wherein the mechanical structure or the gasbag is configured to fix the finger stall on a finger of a user;
   a pulse wave sensor comprising a light emitter and a photodiode, wherein the light emitter is configured to emit a light signal which reaches the photodiode after being reflected or transmitted by a to-be-detected object, and the photodiode is configured to convert the received light signal into an electrical signal;
   an analog front end electrically connected to the pulse wave sensor and configured to receive the electrical signal and output an analog front end signal; and
   a microcontroller unit electrically connected to the analog front end and configured to receive the analog front end signal and output the biometric information, the biometric information comprising a blood pressure value.

2. The apparatus according to claim 1, wherein the apparatus further comprises a pressure detector electrically connected to the analog front end.

3. The apparatus according to claim 1 or 2, wherein the finger stall is a two-end through type finger stall with an annular cross section.

4. The apparatus according to claim 1 or 2, wherein the finger stall is a non-through type;

   the non-through type finger stall is only provided with an opening at one end; and
   a cross section of the non-through type finger stall is one of rectangle, circle, ellipse, and polygon, and the non-through type finger stall has an internal space for accommodating the finger of the user.

5. The apparatus according to claim 3, wherein the finger stall further comprises an air conduit connected to the gasbag.

6. The apparatus according to claim 4, wherein the mechanical structure comprises a spring structure, wherein the spring structure comprises a spring body and a connecting plate; and
   the connecting plate is connected to one end of the spring body, another end of the spring body is connected to the finger stall, and the spring structure is arranged on one side inside the finger stall close to the opening.

7. The apparatus according to claim 6, wherein the spring body comprises a first spring body and a second spring body, and the connecting plate comprises a first connecting plate and a second connecting plate; and
   the first connecting plate is connected to the first spring body, the second connecting plate is connected to the second spring body, the light emitter is arranged on the first connecting plate, and the photodiode is arranged on the second connecting plate.

8. The apparatus according to claim 4, wherein the mechanical structure or the gasbag is arranged on one side inside the finger stall close to the opening.

9. The apparatus according to claim 4, wherein the mechanical structure comprises a motor structure, wherein the motor structure comprises a motor and a connecting plate, the connecting plate is connected to the motor, the motor is configured to control movement of the connecting plate, and the motor structure is arranged on one side inside the finger stall close to the opening.

10. The apparatus according to claim 1, wherein the biometric information further comprises at least one of a heart rate value and a blood oxygen value.

11. An electronic device, comprising the apparatus according to any one of claims 1 to 10 and a display screen arranged on an outer surface of the apparatus, wherein the display screen is configured to display the biometric information.

12. The electronic device according to claim 11, comprising a smart ring, a finger clip-type blood pressure meter, a finger clip-type blood pressure oximeter, and a finger clip-type multi-parameter meter.

FIG. 1

FIG. 2

FIG. 3

(a) (b)

FIG. 4

Parameter1

Parameter2

Parameter3

...

SBP/DBP

FIG. 5

FIG. 6

FIG. 7

FIG. 8

Electronic
device 40

Display screen 30

Apparatus for
detecting biometric
information20

FIG. 9

FIG. 10

## INTERNATIONAL SEARCH REPORT

| International application No. |
|---|
| **PCT/CN2023/102523** |

**A. CLASSIFICATION OF SUBJECT MATTER**

A61B5/021(2006.01)i

According to International Patent Classification (IPC) or to both national classification and IPC

**B. FIELDS SEARCHED**

Minimum documentation searched (classification system followed by classification symbols)

IPC:A61B5

Documentation searched other than minimum documentation to the extent that such documents are included in the fields searched

Electronic data base consulted during the international search (name of data base and, where practicable, search terms used)

CNABS, CNTXT, CNKI, CJFD: 血压, 脉搏, 光电容积, 光电二极管, 光电传感器, 弹, 气囊, 气袋, 气腔, 压力, 手指, 指尖, 指袖, 戒指; VEN, USTXT, WOTXT, EPTXT, IEEE, Elsevier Science, SpringerLink: blood pressure, pulse, photoelectric, PPG, photo diode, photodiode, spring, air chamber, gasbag, airbag, pressure, finger, fingertip, ring

**C. DOCUMENTS CONSIDERED TO BE RELEVANT**

| Category* | Citation of document, with indication, where appropriate, of the relevant passages | Relevant to claim No. |
|---|---|---|
| X | CN 114983364 A (HANGZHOU MEGASENS TECHNOLOGY CO., LTD.) 02 September 2022 (2022-09-02)<br>description, paragraphs 84-102, and figures 1-6 | 1-5, 8-12 |
| Y | CN 114983364 A (HANGZHOU MEGASENS TECHNOLOGY CO., LTD.) 02 September 2022 (2022-09-02)<br>description, paragraphs 84-102, and figures 1-6 | 6, 7, 11, 12 |
| Y | CN 215503017 U (SHENZHEN MERICONN TECHNOLOGY CO., LTD.) 14 January 2022 (2022-01-14)<br>description, paragraphs 26-28, and figures 1-3 | 6, 7, 11, 12 |
| X | CN 110301906 A (SAMSUNG ELECTRONICS CO., LTD.) 08 October 2019 (2019-10-08)<br>description, paragraphs 47-59, and figures 1-4 | 1-5, 8-12 |
| X | KR 20090052442 A (LG ELECTRONICS INC.) 26 May 2009 (2009-05-26)<br>description, paragraphs 18-56, and figures 6-9 | 1-5, 8, 10-12 |
| X | JP S642626 A (OMRON TATEISI ELECTRONICS CO. et al.) 06 January 1989 (1989-01-06)<br>description, the embodiments, and figures 1-5 | 1-5, 8, 10-12 |

☑ Further documents are listed in the continuation of Box C. ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **04 March 2024** | **08 March 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| | International application No. |
|---|---|
| | **PCT/CN2023/102523** |

### C. DOCUMENTS CONSIDERED TO BE RELEVANT

| Category* | Citation of document, with indication, where appropriate, of the relevant passages | Relevant to claim No. |
|---|---|---|
| X | CN 110448287 A (THE CHINESE UNIVERSITY OF HONG KONG et al.) 15 November 2019 (2019-11-15)<br>description, paragraphs 18-49 | 1-5, 8, 10-12 |
| Y | CN 210138143 U (NANJING TANGCHAO TECHNOLOGY CO., LTD.) 13 March 2020 (2020-03-13)<br>description, paragraphs 22-23, and figures 1-5 | 6, 7, 11, 12 |
| Y | CN 111603173 A (SHENZHEN MEIYUNSHENG TECHNOLOGY CO., LTD.) 01 September 2020 (2020-09-01)<br>description, paragraph 24, and figures 1-7 | 6, 7, 11, 12 |
| A | CN 107773223 A (SHANGHAI YOUKEER TECHNOLOGY CO., LTD.) 09 March 2018 (2018-03-09)<br>entire document | 1-12 |
| A | KR 20060081178 A (YONSEI UNIVERSITY, SEOUL) 12 July 2006 (2006-07-12)<br>entire document | 1-12 |

Form PCT/ISA/210 (second sheet) (July 2022)

| | | | |
|---|---|---|---|
| **INTERNATIONAL SEARCH REPORT** | | International application No. | |
| **Information on patent family members** | | **PCT/CN2023/102523** | |

| Patent document cited in search report | | | Publication date (day/month/year) | Patent family member(s) | | | Publication date (day/month/year) |
|---|---|---|---|---|---|---|---|
| CN | 114983364 | A | 02 September 2022 | CN | 114983364 | B | 15 September 2023 |
| | | | | WO | 2023226824 | A1 | 30 November 2023 |
| CN | 215503017 | U | 14 January 2022 | None | | | |
| CN | 110301906 | A | 08 October 2019 | US | 2019298188 | A1 | 03 October 2019 |
| | | | | US | 11576583 | B2 | 14 February 2023 |
| | | | | KR | 20190113543 | A | 08 October 2019 |
| | | | | DE | 102019104568 | A1 | 02 October 2019 |
| KR | 20090052442 | A | 26 May 2009 | CA | 2620546 | A1 | 09 August 2008 |
| | | | | KR | 20080074623 | A | 13 August 2008 |
| | | | | US | 2008214942 | A1 | 04 September 2008 |
| JP | S642626 | A | 06 January 1989 | JPH | 0751123 | B2 | 05 June 1995 |
| CN | 110448287 | A | 15 November 2019 | US | 2019336016 | A1 | 07 November 2019 |
| | | | | US | 11089966 | B2 | 17 August 2021 |
| | | | | CN | 110448287 | B | 04 November 2022 |
| CN | 210138143 | U | 13 March 2020 | None | | | |
| CN | 111603173 | A | 01 September 2020 | CN | 111603173 | B | 05 February 2021 |
| CN | 107773223 | A | 09 March 2018 | None | | | |
| KR | 20060081178 | A | 12 July 2006 | KR | 100660349 | B1 | 21 December 2006 |

Form PCT/ISA/210 (patent family annex) (July 2022)